# EUROPEAN PATENT SPECIFICATION

(11) **EP 2 225 232 B1**
(45) Date of publication and mention of the grant of the patent: **26.09.2012**
(21) Application number: 08738051.5
(22) Date of filing: 30.04.2008
(51) Int. Cl.: C07D 411/04

(54) **PROCESS FOR THE PREPARATION OF SUBSTITUTED 1,3-OXATHIOLANES**
VERFAHREN ZUR HERSTELLUNG SUBSTITUIERTER 1,3-OXATHIOLANE
PROCÉDÉ DE PRÉPARATION DE 1,3-OXATHIOLANES SUBSTITUÉS

(30) Priority: 29.11.2007 IN DE25002007
(43) Date of publication of application: 08.09.2010
(73) Proprietor: Ranbaxy Laboratories Limited, New Delhi 110019 Delhi (IN)
(72) Inventor: TEWARI, Neera, Haryana 122001 (IN); NAIR, Dinesh, Shashidharan, Gurjarat 390023 (IN); MEERAN, Hashim, Nizar, Poovanathil, Nagoor, Pathanamthitta Kerala 689645 (IN); PRASAD, Mohan, Haryana 122001 (IN)
(74) Representative: Cronin, Brian Harold John
(86) International application number: PCT/IB2008/051689
(87) International publication number: WO 2009/069011

(56) References cited:
- WO-A-00/64427
- US-A- 5 693 787
- US-A- 6 113 920
- US-B1- 6 329 522
- GOODYEAR ET AL: "Practical enantioselective synthesis of lamivudine (3TC<(>TM)) via a dynamic kinetic resolution" TETRAHEDRON LETTERS, ELSEVIER, AMSTERDAM, vol. 46, no. 49, 5 December 2005 (2005-12-05), pages 8535-8538, XP005171940 ISSN: 0040-4039 cited in the application

## Description

### Field of the Invention

The present invention relates to a process for the preparation of substituted 1,3-oxathiolanes. The present invention specifically relates to a process for the preparation of lamivudine.

### Background of the Invention

Substituted 1,3-oxathiolanes of Formula I and stereoisomers thereof, 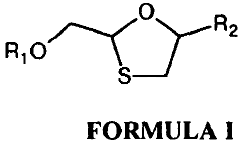 wherein R₁ is hydrogen, alkyl or aryl, and R₂ is a substituted or unsubstituted purine or pyrimidine base or an analogue or derivative thereof, are an important class of therapeutic agents and they have shown antiviral activity against retroviruses such as human immunodeficiency virus (HIV), hepatitis B virus (HBV) and human T-lymphotropic virus (HTLV). Lamivudine is a substituted 1,3-oxathiolane and it is presently available in the market as an antiretroviral agent. Lamivudine is the cis-(-)-isomer and it is chemically (2R,cis)-4-amino-1-(2-hydroxymethyl-1,3-oxathiolan-5-yl)-(1H)-pyrimidin-2-one as represented by Formula I (A).

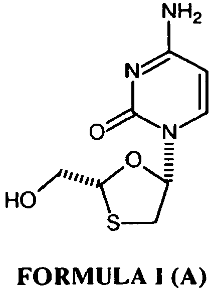

Two different approaches have been reported for preparing substituted 1,3-oxathiolanes by using specific leaving groups and Lewis acid catalysts.

The first approach involves condensing an intermediate of Formula II, or its stereoisomers thereof 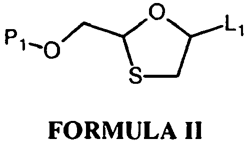 wherein P₁ is a protecting group and L₁ is OCH₃, OC₂H₅ or OCOCH₃, with a silyl and/or acetyl protected pyrimidine or purine base. The condensed product is finally deprotected to obtain desired substituted 1,3-oxathiolanes. This approach is provided in U.S. Patent Nos. 5,047,407 and 5,905,082, J. Org. Chem., (1992), 57:2217-2219, J. Med. Chem., (1993), 36:181-195, and J. Org. Chem., (1991), 56:6503-6505. These references describe processes wherein the condensation is carried out in the presence of silyl Lewis acids such as trimethylsilyl triflate. However, this approach does not provide optically pure 1,3-oxathiolanes and preparation of lamivudine by this approach results in a mixture of at least two of the following isomers.

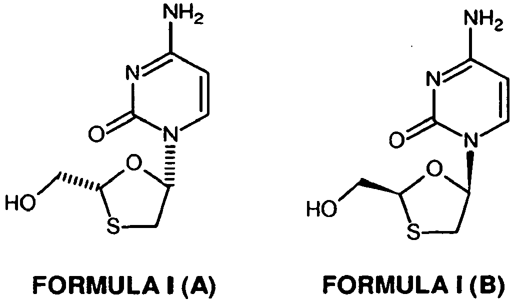

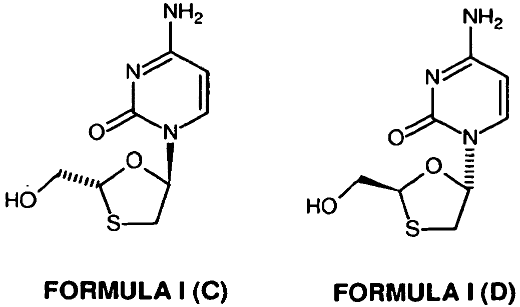

The prior art references mentioned above employ chiral chromatography, or enzymatic resolution to isolate lamivudine of Formula I (A) from said mixture. Synthetic Communications, (2002), 32:2355-2359 provides a separation method for lamivudine using a chiral auxiliary from its mixture with the compound of Formula I (B). U.S. Patent No. 5,204,466 employs stannic chloride instead of silyl Lewis acids in the condensation of the intermediate of Formula II with silylated cytosine. However, J. Org. Chem., (1992), 57:2217-2219 says that the use of stannic chloride as a catalyst also results in a racemic mixture based on optical rotation and chiral HPLC analysis of the product obtained.

The second approach involves condensing an intermediate of Formula III, or its stereoisomers thereof 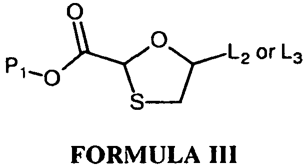 wherein P₁ is a protecting group, L₂ is OCOCH₃, L₃ is halo, with a silyl and/or acetyl protected pyrimidine or purine base. The condensed product is finally reduced and deprotected to obtain desired substituted 1,3-oxathiolanes. U.S. Patent No. 5,663,320 provides a method to carry out the condensation in the presence of silyl Lewis acids such as iodotrimethyl silane. U.S. '320 patent employs the compound of Formula III with OCOCH₃ group at 5-position (L₂) as an intermediate. U.S. '320 patent further says that the choice of silyl Lewis acids is the key feature of the above process. However, it has been reported in Tetrahedron Letters, (2005), 46:8535-8538 that the approach involving iodotrimethyl silane is proved to be inefficient for preparing lamivudine as it is low yielding and requires selective crystallization of the intermediates to obtain desired optical purity and so is low yielding. To overcome such purity and yield issues related to Lewis acid catalysts, Tetrahedron Letters, (2005), 46:8535-8538 and U.S. Patent No. 6,329,522 provide a method to carry out the condensation in the absence of any Lewis acid catalyst by selectively using the compound of Formula III with a halo group at 5-position (L₃) as an intermediate. However, this method requires stereoselective synthesis of the compound of Formula III with halo group at 5-position and the condensation step requires reflux temperature conditions in a high boiling solvent such as toluene.

U.S. Patent No. 6,329,522 also provides a method to purify lamivudine by preparing the salicylate salt of lamivudine. The process involves dissolving a mixture of lamivudine with excess of salicylic acid in water by heating at 71°C, cooling to 58°C and seeding with standard lamivudine salicylate. The solution of lamivudine salicylate seeded with authentic lamivudine salicylate is cooled to 5° to 10°C and stirred at this temperature for 4 hours to obtain solid lamivudine salicylate. The lamivudine salicylate is isolated by filtration and treated with triethylamine in the presence of industrial methylated spirit. The solution is concentrated and the concentrated solution is seeded. The seeded mixture is treated with isopropyl acetate to obtain lamivudine. However, this method requires heating the reaction mixture to 70°C to 75°C while treating with triethylamine and while seeding. Further, US '522 does not disclose or suggest any method to obtain the seed of lamivudine salicylate or lamivudine base. U.S. '522 patent also does not disclose the quantities of seeds to be used in the process. Further, U.S. '522 patent does not provide any method for efficient removal of the residual salicylic acid from lamivudine.

### Summary of the Invention

We have surprisingly found that substituted 1,3-oxathiolanes, such as lamivudine, can be prepared with high optical and chemical purity by using non-silyl Lewis acids in the condensation step. This process also provides a way to obtain substituted 1,3-oxathiolanes with better yield. A chiral auxiliary may need to be used in executing the process but the use of optically pure intermediates and high temperature conditions in the condensation step are not required. The present process can be carried out at a temperature of about 50°C or less. We have also developed an advantageous method for purifying lamivudine by preparing lamivudine salicylate without seeding at any stage. Further, the salt purification step of the present invention can be carried out at temperature conditions of about 55°C or below. The process reported herein also provides pure lamivudine, which is substantially free of salicylic acid. The present process is also suitable to prepare lamivudine at industrial scale.

The term "purine or pyrimidine base or an analogue or derivative thereof" in the present invention refers to a purine or pyrimidine base, which may be found in native nucleosides or synthetic nucleotides that mimic or are derived from such bases in their structures, which may either possess certain additional functional properties of native bases or lack certain functional properties of native bases. The analogues or derivatives include but are not limited to those compounds derived by replacement of a CH₂ moiety by a nitrogen atom or replacement of a nitrogen atom by a CH₂ moiety, or both, or those compounds wherein the ring substituents are either incorporated, removed or modified by conventional substituents known in the art. The functional groups of purine or pyrimidine base or an analogue or derivative thereof may also be protected with hydroxy, amino or carboxyl protecting groups.

The term "protecting group" in the present invention refers to those known and used in the art and serve the function of blocking the carboxyl, amino or hydroxyl groups while the reactions are carried out at other sites of the molecule. Examples of a carboxyl protecting group include, but are not limited to, substituted or unsubstituted alkyl, substituted or unsubstituted alkenyl, substituted or unsubstituted aralkyl, substituted or unsubstituted aryl, substituted or unsubstituted amino, hydrocarbonated silyl, hydrocarbonated stannyl, and a pharmaceutically active ester forming group. Examples of hydroxyl and amino protecting groups include, but are not limited to, lower alkylsilyl groups, lower alkoxymethyl groups, aralkyl groups, acyl groups, lower alkoxycarbonyl groups, alkenyloxycarbonyl groups and aralkyloxycarbonyl groups. Examples of amino protecting groups include but are not limited to alkylidene groups substituted with optionally protected hydroxy groups. The hydroxyl or carboxyl protecting groups can also be chiral auxiliaries, which may possess one or more chiral centers.

The term "leaving group" in the present invention refers to an atom or a group which is displaceable upon reaction with a purine or pyrimidine base or an analogue or derivative thereof. Examples of leaving groups include but are not limited to substituted or unsubstituted, saturated or unsaturated acyloxy groups, alkoxy groups, alkoxy carbonyl groups, halogens, amido, azido, cyano, isocyanato, substituted or unsubstituted, saturated or unsaturated thiolates, and substituted or unsubstituted seleno, seleninyl or selenonyl. Examples of leaving groups also include but are not limited to - OX, wherein X is substituted or unsubstituted aryl, heteroaryl, phosphonate, sulfinyl or sulfonoyl group.

### Detailed Description of the Invention

A first aspect of the present invention provides a process for the preparation of a substituted 1,3-oxathiolane of Formula I or its stereoisomers, and salts thereof, 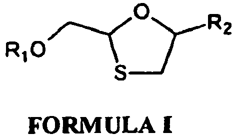 wherein R₁ is hydrogen, alkyl or aryl, and R₂ is a substituted or unsubstituted purine or pyrimidine base or an analogue or derivative thereof,
wherein the process comprises,
a) reacting a compound of Formula III or its stereoisomers thereof, 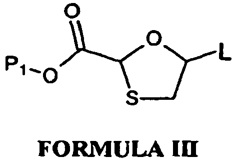 wherein P₁ is hydrogen or a protecting group and L is a leaving group, with a substituted or unsubstituted purine or pyrimidine base as defined below, in the presence of a Lewis acid with the proviso that the Lewis acid does not contain any silyl group, to obtain a compound of Formula IV or its stereoisomers thereof, 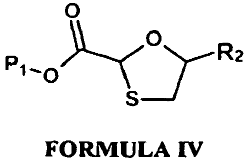 wherein P₁ is hydrogen or a protecting group and R₂ is a substituted or unsubstituted purine or pyrimidine base or an analogue or derivative thereof,
b) reducing the compound of Formula IV or its stereoisomers thereof to obtain the compound of Formula I or stereoisomers thereof, and
c) isolating the compound of Formula I or its stereoisomers, and salts thereof, from the reaction mixture thereof.

The compound of Formula III or its stereoisomers thereof can be prepared according to the methods provided in U.S. Patent No. 5,663,320 or Tetrahedron Letters, (2005), 46:8535-8538. The compound of Formula III may be used as a single isomer or as a mixture of two or more isomers. The leaving group L of the compound of Formula III is preferably selected from the group consisting of acyloxy groups, alkoxy groups, and alkoxy carbonyl groups. The leaving group is more preferably acetyloxy group. The compound of Formula III is reacted with a substituted or unsubstituted purine or pyrimidine base or an analogue or derivative thereof, in the presence of a Lewis acid with the provisothat the Lewis acid does not contain any silyl group. The purine or pyrimidine base or an analogue or derivative thereof is selected from the group consisting of: 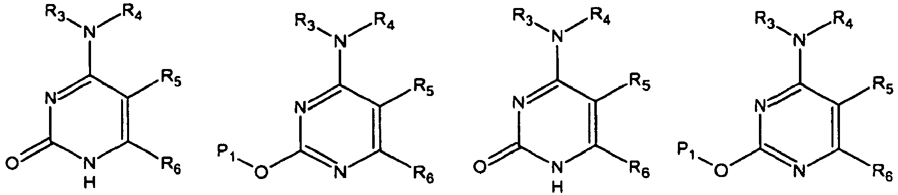 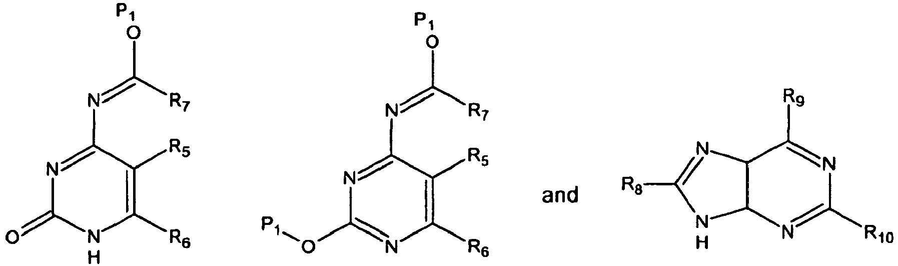 wherein P₁ is a protecting group, R₃ and R₄ are independently selected from the group consisting of hydrogen, hydroxyl, amino, and substituted or unsubstituted C₁₋₆ alkyl, C₁₋₆ alkenyl, C₁₋₆ alkynyl, C₁₋₆ acyl or aracyl; R₅ and R₆ are independently selected from the group consisting of hydrogen, halogen, hydroxyl, amino, cyano, carboxy, carbamoyl, alkoxycarbonyl, hydroxymethyl, trifluoromethyl, thioaryl, and substituted or unsubstituted C₁₋₆ alkyl, C₁₋₆ alkenyl, C₁₋₆ alkynyl, or C₁₋₁₀ acyloxy; R₇ is C₁₋₆ alkyl, C₁₋₆ alkenyl, or C₁₋₆ alkynyl; R₈ is selected from the group consisting of hydrogen, hydroxy, alkoxy, thiol, thioalkyl, substituted or unsubstituted amino, halogen, cyano, carboxy, alkoxycarbonyl, carbamoyl, and substituted or unsubstituted C₁₋₆ alkyl, C₁₋₆ alkenyl, C₁₋₆ alkynyl, or C₁₋₁₀ acyloxy; and R₉ and R₁₀ is selected from the group consisting of hydrogen, hydroxy, alkoxy, substituted or unsubstituted amino, halogen, azido, and substituted or unsubstituted C₁₋₆ alkyl, C₁₋₆ alkenyl, C₁₋₆ alkynyl, or C₁₋₁₀ acyloxy.

The Lewis acid is preferably stannic chloride or titanium tetrachloride. The Lewis acid is used in about 0.5 to about 1.5 molar equivalents to the quantity of the compound of Formula III. The Lewis acid is preferably used in about 0.8 to about 1.1 molar equivalents. The reaction is carried out in the presence of an organic solvent selected from the group consisting of aliphatic hydrocarbons, aromatic hydrocarbons, halogenated hydrocarbons, nitriles, amides, esters, and ketones. The reaction is preferably carried out at a temperature of about 50°C or below. The reaction is carried out for about 10 minutes to about 100 hours. The compound of Formula IV or its stereoisomers can be isolated from the reaction mixture or directly used in the subsequent step without isolation. The compound of Formula IV or its stereoisomers are preferably isolated from the reaction mixture. The compound of Formula IV or its stereoisomers may be subjected to purification to remove chemical impurities and/or undesired isomers. The protecting groups, if any, present in the compound of Formula IV can be removed and the deprotected compound reduced to obtain the compound of Formula I or its stereoisomers. The reduction is carried out by using a reducing agent. The reducing agent can be, for example, sodium borohydride, lithium aluminium hydride or lithium borohydride. The compound of Formula I or its stereoisomers can be further purified by salt formation, crystallization, isomer separation or chromatographic methods or a combination thereof.

A second aspect of the present invention provides a process for the preparation of lamivudine of Formula I (A) or a compound of Formula I (C), or mixtures thereof, 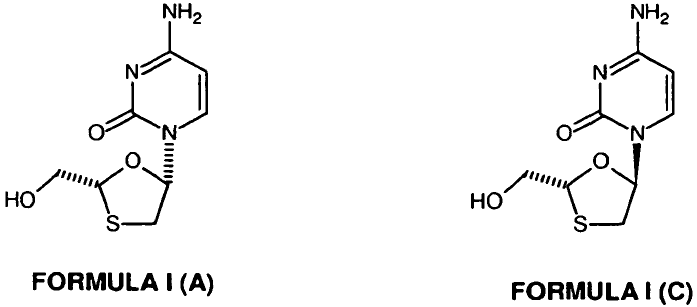 wherein the process comprises,
a) reacting a compound of Formula III (A) or Formula III (B), or mixtures thereof, 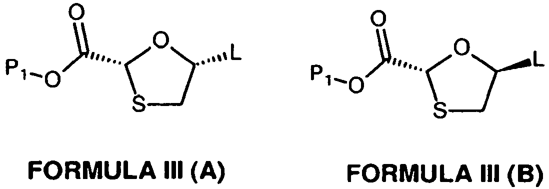 wherein P₁ is a chiral auxiliary and L is a leaving group, with cytosine, wherein the amino or hydroxy, or both the groups of said cytosine are optionally protected with protecting groups, in the presence of a Lewis acid with the provisothat the Lewis acid does not contain any silyl group, to obtain a compound of Formula IV (A) or Formula IV (B), or mixtures thereof 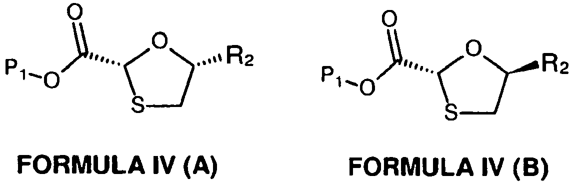 wherein P₁ is a chiral auxiliary and R₂ is cytosine, wherein the amino or hydroxy, or both the groups of said cytosine are optionally protected with protecting groups,
b) reducing the compound of Formula IV (A) or Formula IV (B), or mixtures thereof, to obtain lamivudine of Formula I (A) or the compound of Formula I (C), or mixtures thereof, and
c) isolating lamivudine of Formula I (A) or the compound of Formula I (C), or mixtures thereof, from the reaction mixture thereof.

The compound of Formula III (A) or Formula III (B), or mixtures thereof, can be prepared according to the methods provided in U.S. Patent No. 5,663,320 or Tetrahedron Letters, (2005), 46:8535-8538. The leaving group L of the compound of Formula III (A) or Formula III (B), or mixtures thereof, is preferably selected from the group consisting of acyloxy groups, alkoxy groups, and alkoxy carbonyl groups. The leaving group is more preferably acetyloxy group. The chiral auxiliary P₁ of the compound of Formula III (A) or Formula III (B), or mixtures thereof, is preferably an L-menthyl group. The compound of Formula III (A) or Formula III (B), or mixtures thereof is reacted with cytosine, wherein the amino or hydroxy, or both the groups of cytosine are optionally protected with protecting groups. The cytosine is preferably protected with acetyl and/or silyl protecting groups. The reaction is carried out in the presence of a Lewis acid with the provisothat the Lewis acid does not contain any silyl group. The Lewis acid is preferably stannic chloride or titanium tetrachloride. The Lewis acid is used in about 0.5 to about 1.5 molar equivalents to the quantity of the compound of Formula III. The Lewis acid is preferably used in about 0.8 to about 1.1 molar equivalents. The reaction is carried out in the presence of an organic solvent selected from the group consisting of aliphatic hydrocarbons, aromatic hydrocarbons, halogenated hydrocarbon, nitriles, amides, esters, and ketones, The reaction is preferably carried out at a temperature of about 50°C or below. The reaction is carried out for about 10 minutes to about 100 hours.

The compound of Formula IV (A) or Formula IV (B), or mixtures thereof can be isolated from the reaction mixture or directly used in the subsequent step without isolation. The compound of Formula IV (A) or Formula IV (B), or mixtures thereof are preferably isolated from the reaction mixture. A deprotection step may be carried out to remove the protecting groups, if any, present in R₂ of the compound of Formula IV (A) or Formula IV (B), or mixtures thereof. The compound of Formula IV (A) or Formula IV (B), or mixtures thereof may be subjected to purification to remove chemical impurities and/or undesired isomers. The compound of Formula IV (A) or Formula IV (B), or mixtures thereof are reduced to obtain lamivudine of Formula I (A) or the compound of Formula I (C), or mixtures thereof. The reduction is carried out by using a reducing agent. The reducing agent can be, for example, sodium borohydride, lithium aluminium hydride, lithium borohydride, lithium-tri-ethyl borohydride or lithium-tri-sec-butyl borohydride. Lamivudine of Formula I (A) or the compound of Formula I (C), or mixtures thereof can be further purified by salt formation, crystallization, isomer separation or chromatographic methods or a combination thereof.

A third aspect of the present invention provides a process for the preparation of lamivudine of Formula I (A), 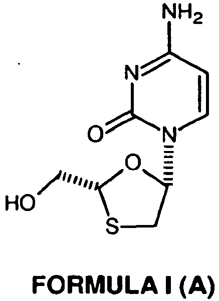 wherein the process comprises,
a) reacting a compound of Formula III (C), 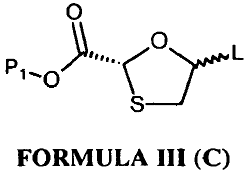 wherein **P₁** is a chiral auxiliary and L is a leaving group, with cytosine, wherein the amino or hydroxy, or both the groups of said cytosine are optionally protected with protecting groups, in the presence of a Lewis acid with the provisothat the Lewis acid does not contain any silyl group, to obtain a compound of Formula IV (C), 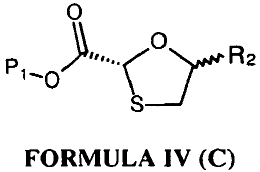 wherein P₁ is a chiral auxiliary and R₂ is cytosine, wherein the amino or hydroxy, or both the groups of said cytosine are optionally protected with protecting groups,
b) separating a compound of Formula IV (A) from the reaction mixture thereof, 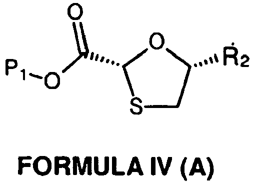 wherein P₁ is a chiral auxiliary and R₂ is cytosine, wherein the amino or hydroxy, or both the groups of said cytosine are optionally protected with protecting groups,
c) reducing the compound of Formula IV (A), and
d) isolating lamivudine of Formula I (A) from the reaction mixture thereof.

The compound of Formula III (C) can be prepared according to the methods provided in U.S. Patent No. 5,663,320 or Tetrahedron Letters, (2005), 46:8535-8538. The leaving group L of the compound of Formula III (C) is preferably selected from the group consisting of acyloxy groups, alkoxy groups, and alkoxy carbonyl groups. The leaving group is more preferably acetyloxy group. The chiral auxiliary P₁ of the compound of Formula III (C) is preferably an L-menthyl group. The compound of Formula III (C) is reacted with cytosine, wherein the amino or hydroxy, or both the groups of said cytosine are optionally protected with protecting groups. The cytosine is preferably protected with acetyl and/or silyl protecting groups. The reaction is carried out in the presence of a Lewis acid with the provisothat the Lewis acid does not contain any silyl group. The Lewis acid is preferably stannic chloride or titanium tetrachloride. The Lewis acid is used in about 0.5 to about 1.5 molar equivalents to the quantity of the compound of Formula III. The Lewis acid is preferably used in about 0.8 to about 1.1 molar equivalents. The reaction is carried out in the presence of an organic solvent selected from the group consisting of aliphatic hydrocarbons, aromatic hydrocarbons, halogenated hydrocarbon, nitriles, amides, esters, and ketones. The reaction is preferably carried out at a temperature of about 50°C or below. The reaction is carried out for about 10 minutes to about 100 hours.

The compound of Formula IV (C) so obtained may be subjected to deprotection to remove the silyl protecting groups, if any, present in the cytosine group. The compound of Formula IV (C) is isolated from the reaction mixture by concentrating the reaction mixture. The compound of Formula IV (A) is separated from the compound of Formula IV (C) by selective crystallization methods, chiral chromatographic methods or by chiral salt formation, or a combination thereof. The compound of Formula IV (A) is preferably separated by treating the compound of Formula IV (C) with a solvent, which selectively dissolves the undesired isomers while the compound of Formula IV (A) is partially or completely insoluble in said solvent. The treatment with the solvent may be carried out once or more than once to achieve desired optical purity. The solvent is preferably a C₁₋₃ alkanol or an aliphatic ester, or a mixture thereof, more preferably methanol or isopropyl acetate, or a mixture thereof. The compound of Formula IV (A) may be isolated from the reaction mixture by filtration after treating with the solvent.

The compound of Formula IV (A) is deprotected to remove the acetyl protecting groups, if any, present in R₂ of the compound of Formula IV (A), and reduced to obtain lamivudine of Formula I (A). The reduction is carried out by using a reducing agent. The reducing agent can be, for example, sodium borohydride, lithium aluminium hydride, lithium borohydride, lithium-tri-ethyl borohydride or lithium-tri-sec-butyl borohydride. The reducing agent is preferably sodium borohydride. The reduction is carried out in the presence of a phosphate or borate buffer. The buffer is preferably dipotassium hydrogen phosphate. Lamivudine of Formula I (A) can be further purified by salt formation, crystallization, or chromatographic methods, or a combination thereof.

### EXAMPLES

### Example 1: Preparation of (1R,2S,5R)-2-isopropyl-5-methylcyclohexyl (2R,5S)-5-[4-(acetylamino)-2-oxopyrimidin-1(2H)-yl]-1,3-oxathiolane-2-carboxylate

N-Acetyl cytosine (100 g) was added to xylene (200 mL), followed by the addition of hexamethyldisilazane (200 mL) and trimethylchlorosilane (5.0 mL). The reaction mixture was heated in at 140° to 145°C for 3 to 4 hours. The reaction mixture was cooled to 100°C and xylene (200 mL) was added to the reaction mixture. The reaction mixture was distilled under vacuum at 120° to 130°C. Xylene (200 mL) was added to the reaction mixture and recovered under vacuum to obtain a residue. Dichloroethane (2 L) was added to the residue, followed by the addition of (2*S*,S*R*)-2-isopropyl-5-methylcyclohexyl (2*R*)-5-(acetyloxy)-1,3-oxathiolane-2-carboxylate (194 g). Stannic chloride (152 g) was added drop-wise to the reaction mixture at 40° to 45°C and stirred for 5 hours at 40° to 45°C. The reaction mixture was cooled to about 25°C, followed by the addition of a mixture of methanol (1L) and water (1 L). The reaction mixture was allowed to settle and the organic layer was added to a mixture of water (1 L) and methanol (1 L). The reaction mixture was stirred for 5 to 10 minutes and allowed to settle. The organic layer was concentrated under vacuum at 40° to 50°C. Isopropyl acetate (1.5 L) was added to the resultant mass and stirred overnight at about 25°C. The reaction mixture was filtered, washed with isopropyl acetate (2 X 100 mL) and dried at 45° to 50°C for 5 hours. The solid so obtained was suspended in methanol (500 mL) at about 25°C and stirred for 3 hours at the same temperature. The mixture was filtered, washed with methanol (100 mL) and dried at 45° to 50°C for 5 hours to obtain the title compound.
Yield: 28 g

### Example 2: Preparation of (1R,2S,5R)-2-isopropyl-5-methylcyclohexyl (2R,5S)-5-(4-amino-2-oxopyrimidin-1(2H)-yl)-1,3-oxathiolane-2-carboxylate

(1*R*,2*S*,5*R*)-2-isopropyl-5-methylcyclohexyl (2*R*,5*S*)-5-[4-(acetylamino)-2-oxopyrimidin-1(2*H*)-yl]-1,3-oxathiolane-2-carboxylate (100 g) obtained from Example 1 was suspended in methanol (600 mL) at about 25°C. Methane sulphonic acid (29.4 g) was added drop-wise to the suspension in 15 to 20 minutes at 25° to 30°C and stirred for 4 hours at about 25°C. The reaction mixture was added slowly to a mixture of dichloromethane (1 L) and aqueous sodium bicarbonate solution (28 g of sodium bicarbonate dissolved in 1.2 L of water.) The reaction mixture was stirred for 5 to 10 minutes and allowed to settle. The layers were separated and the aqueous layer was reextracted with dichloromethane (250 mL). The organic layers were combined and concentrated, followed by the addition of hexane (500 mL). The reaction mixture was stirred for about 2 hours and filtered. The solid was washed with hexane (100 mL) and subsequently with isopropyl acetate (200 mL), and dried at 45° to 50°C to obtain the title compound.
Yield: 82 g

### Example 3: Preparation of Lamivudine Salicylate

Dipotassium hydrogen orthophosphate (205.5 g) was added to deionised water (423 mL) and stirred at 25° to 30°C to obtain a solution. The solution was cooled to 17° to 22°C, followed by the addition of denaturated spirit (900 mL) at the same temperature and stirred for 5 minutes. (1*R*,2*S*,5*R*)-2-isopropyl-5-methylcyclohexyl (2*R*,5*S*)-5-(4-amino-2-oxopyrimidin-1(2*H*)-yl)-1,3-oxathiolane-2-carboxylate (150 g) obtained from Example 2 was added to the mixture at 17° to 22°C and stirred for 30 minutes at 18° to 20°C. Sodium borohydride solution was added slowly to the reaction mixture over a period of 2 to 3 hours at 18° to 20°C. (Preparation of sodium borohydride solution: Sodium hydroxide (0.75 g) was dissolved in deionised water (143 mL). Sodium borohydride (30 g) was added to the solution at 20° to 35°C, stirred at 20° to 35°C to obtain a solution and cooled to 17° to 22°C). The reaction mixture was stirred for 6 hours at 18° to 22°C and the reaction mixture was allowed to settle at 18° to 25°C. The organic layer was separated and denaturated spirit (150 mL) was added to the aqueous layer at 18° to 25°C. The reaction mixture was stirred for 15 minutes at the same temperature and allowed to settle. The organic layer was separated and combined with the previously obtained organic layer. The pH of the combined organic layer was adjusted to 6.0 to 6.5 with dilute hydrochloric acid (20 mL; prepared by mixing 10 mL of concentrated hydrochloric acid with 10 mL of deionised water) at 18° to 25°C, followed by stirring for 10 minutes at the same temperature. The pH of the reaction mixture was adjusted to 8.0 to 8.5 with aqueous sodium hydroxide solution (28 mL; prepared by dissolving 2.1 g of sodium hydroxide in 27 mL of deionised water) at 18° to 25°C. The reaction mixture was concentrated under vacuum at about 55°C till the residual volume was about 375 mL. Deionised water (300 mL) was added to the concentrated reaction mixture at 25° to 30°C and stirred for 10 minutes. The reaction mixture was washed with toluene (2 X 150 mL) at 25° to 30°C and the toluene layer was extracted with deionised water (150 mL) at 25° to 30°C. The aqueous layers were combined and salicylic acid (57 g) was added at 25° to 30°C. Deionised water (150 mL) was added to the reaction mixture and heated to 78° to 82°C to get a clear solution. The reaction mixture was cooled to 25° to 30°C over a period of 2 hours and stirred at the same temperature for 4 hours. The reaction mixture was further cooled to 10° to 15°C and stirred for 2 hours at 10° to 15°C. The solid was filtered, washed with deionised water (150 mL) and dried by suction. The solid so obtained was washed with methanol (90 mL, pre-cooled to 5° to 10°C) and dried at 45° to 50°C in hot air oven to obtain the title compound.
Yield: 132 g

### Example 4: Preparation of Lamivudine

Lamivudine salicylate (120 g) obtained from Example 3 was added to a mixture of ethyl acetate (720 mL) and water (6 mL) at 25° to 35°C. The reaction mixture was heated to 45° to 50°C, followed by the addition of triethylamine (104.76 g) over 30 minutes at 45° to 50°C. The reaction mixture was stirred for 4 hours at the same temperature and cooled to 25° to 30°C. The reaction mixture was stirred for further 30 minutes at 25° to 30°C, filtered and dried by suction. The solid obtained was washed with ethyl acetate. Ethyl acetate (600 mL) was added to the washed solid and heated to 50° to 55°C. The mixture was stirred at 50° to 55°C for 15 minutes, cooled to 25° to 30°C and stirred for further 30 minutes. The solid was filtered at 25° to 30°C, washed with ethyl acetate (60 mL) and dried under vacuum at 45° to 50°C to obtain the title compound.
Yield: 68.5 g

### reference Example 5: Purification of Lamivudine

Lamivudine (60 g) obtained from Example 4 was added to absolute alcohol (1.2 L) at 25° to 35°C. The reaction mixture was heated to 75° to 78°C and stirred to obtain a solution. Activated carbon (6 g) was added to the solution so obtained at 75° to 78°C, stirred for 30 minutes at the same temperature and filtered through Celite bed at the same temperature. The carbon bed was washed with absolute alcohol (60 mL; preheated to 75° to 76°C) and the reaction mixture was concentrated under vacuum to obtain a volume of about 300 mL. The concentrated reaction mixture was heated to 74° to 76°C, stirred for 15 minutes and cooled to 20° to 25°C in 1 hour time. The reaction mixture was further cooled to 5° to 10°C in 1 hour time and stirred for 30 minutes. The solid was filtered, washed with absolute alcohol (30 mL, pre-cooled to 5° to 10°C) and dried under vacuum at 50° to 55°C to obtain the title compound.
Yield: 53 g
HPLC Purity: 99.0%
Chiral Purity: 99.8%
Salicylic acid content (HPLC): Not detectable

## Claims

1. A process for the preparation of a substituted 1,3-oxathiolane of Formula I or its stereoisomers, and salts thereof, 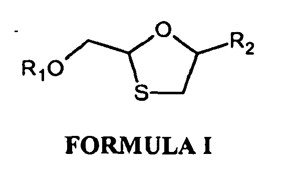 wherein R₁ is hydrogen, alkyl or aryl, and R₂ is a substituted or unsubstituted purine or pyrimidine base selected from the group consisting of: 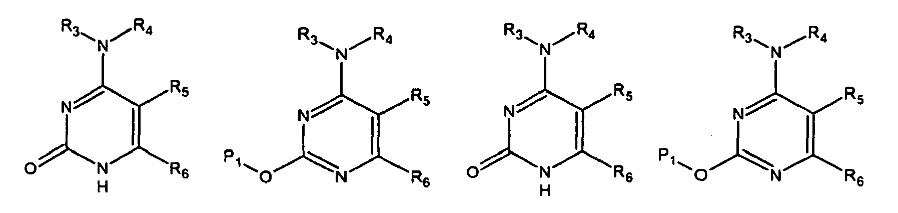 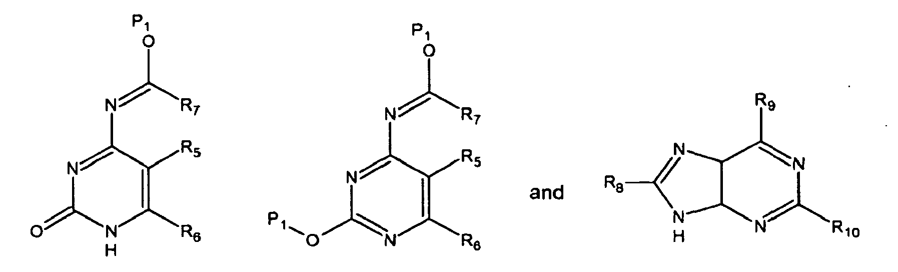 wherein P₁ is a protecting group, R₃ and R₄ are independently selected from the group consisting of hydrogen, hydroxyl, amino, and substituted or unsubstituted C₁₋₆ alkyl, C₁₋₆ alkenyl, C₁₋₆ alkynyl, C₁₋₆ acyl or aracyl; R₅ and R₆ are independently selected from the group consisting of hydrogen, halogen, hydroxyl, amino, cyano, carboxy, carbamoyl, alkoxycarbonyl, hydroxymethyl, trifluoromethyl, thioaryl, and substituted or unsubstituted C₁₋₆ alkyl, C₁₋₆ alkenyl, C₁₋₆ alkynyl, or C₁₋₁₀ acyloxy; R₇ is C₁₋₆ alkyl, C₁₋₆ alkenyl, or C₁₋₆ alkynyl; R₈ is selected from the group consisting of hydrogen, hydroxy, alkoxy, thiol, thioalkyl, substituted or unsubstituted amino, halogen, cyano, carboxy, alkoxycarbonyl, carbamoyl, and substituted or unsubstituted C₁₋₆ alkyl, C₁₋₆ alkenyl, C₁₋₆ alkynyl, or C₁₋₁₀ acyloxy; and R₉ and R₁₀ is selected from the group consisting of hydrogen, hydroxy, alkoxy, substituted or unsubstituted amino, halogen, azido, and substituted or unsubstituted C₁₋₆ alkyl, C₁₋₆ alkenyl, C₁₋₆ alkynyl, or C₁₋₁₀ acyloxy, wherein the process comprises,
a) reacting a compound of Formula III or its stereoisomers thereof, 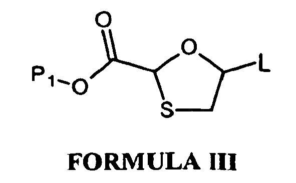 wherein P₁ is hydrogen or a protecting group and L is a leaving group, with a substituted or unsubstituted purine or pyrimidine base defined above, in the presence of a Lewis acid with the proviso that the Lewis acid does not contain any silyl group, to obtain a compound of Formula IV or its stereoisomers thereof, 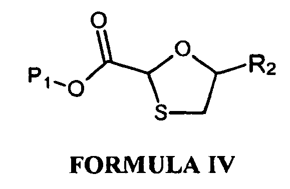 wherein P₁ is hydrogen or a protecting group and R₂ is a substituted or unsubstituted purine or pyrimidine base defined above,
b) reducing the compound of Formula IV or its stereoisomers thereof to obtain the compound of Formula I or stereoisomers thereof, and
c) isolating the compound of Formula I or its stereoisomers, and salts thereof, from the reaction mixture thereof.

2. A process according to claim 1, wherein the Lewis acid is stannic chloride or titanium tetrachloride.

3. A process according to claim 2, wherein the Lewis acid is stannic chloride.

4. A process according to claim 1, wherein step b) is carried out by using a reducing agent.

5. A process according to claim 1, wherein the compound of Formula I is lamivudine.

6. A process for the preparation of lamivudine of Formula I (A) or a compound of Formula I (C), or mixtures thereof 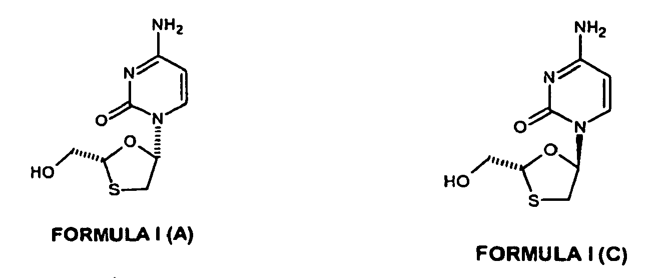 wherein the process comprises,
a) reacting a compound of Formula III (A) or Formula III (B), or mixtures thereof, 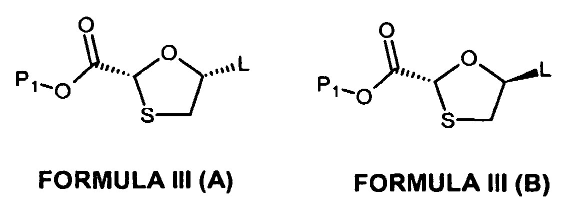 wherein P₁ is a chiral auxiliary and L is a leaving group, with cytosine, wherein the amino or hydroxy, or both the groups of said cytosine are unprotected or protected with protecting groups, in the presence of a Lewis acid with the proviso that the Lewis acid does not contain any silyl group, to obtain a compound of Formula IV (A) or Formula IV (B), or mixtures thereof 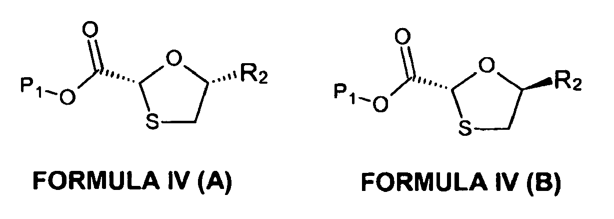 wherein P₁ is a chiral auxiliary and R₂ is cytosine, wherein the amino or hydroxy, or both the groups of said cytosine are optionally protected with protecting groups,
b) reducing the compound of Formula IV (A) or Formula IV (B), or mixtures thereof, to obtain lamivudine of Formula I (A) or the compound of Formula I (C), or mixtures thereof, and
c) isolating lamivudine of Formula I (A) or the compound of Formula I (C), or mixtures thereof, from the reaction mixture.

7. A process according to claim 6, wherein the chiral auxiliary P₁ of the compound of Formula III (A) or Formula III (B), or mixtures thereof, and that of the compound of Formula IV (A) or Formula IV (B), or mixtures thereof is an L-menthyl group.

8. A process according to claim 6, wherein the cytosine is protected with acetyl and/or silyl protecting groups.

9. A process according to claim 6, wherein the Lewis acid is stannic chloride or titanium tetrachloride.

10. A process according to claim 9, wherein the Lewis acid is stannic chloride.

11. A process for the preparation of Lamivudine of Formula I (A), 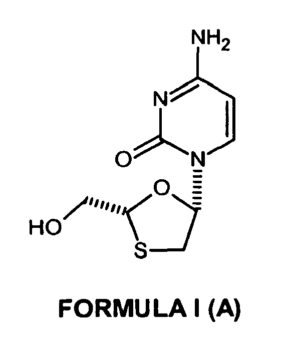 wherein the process comprises,
a) reacting a compound of Formula III (C), 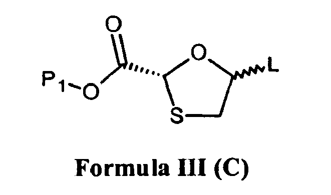 wherein P₁ is a chiral auxiliary and L is a leaving group, with cytosine, wherein the amino or hydroxy, or both the groups of said cytosine are unprotected or protected with protecting groups, in the presence of a Lewis acid with the proviso that the Lewis acid does not contain any silyl group, to obtain a compound of Formula IV (C), 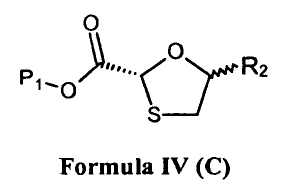 wherein P₁ is a chiral auxiliary and R₂ is cytosine, wherein the amino or hydroxy, or both the groups of said cytosine are unprotected or protected with protecting groups,
b) separating a compound of Formula IV (A) from the reaction mixture thereof, 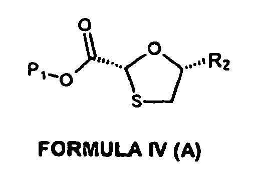 wherein P₁ is a chiral auxiliary and R₂ is cytosine, wherein the amino or hydroxy, or both the groups of said cytosine are unprotected or protected with protecting groups,
c) reducing the compound of Formula IV (A), and
d) isolating lamivudine of Formula I (A) from the reaction mixture thereof.

12. A process according to claim 11, wherein the Lewis acid is stannic chloride or titanium tetrachloride.

13. A process according to claim 12, wherein the Lewis acid is stannic chloride.

14. A process according to claim 11, wherein the compound of Formula IV (A) is separated in step b) by treating the compound of Formula IV (C) with a solvent, which selectively dissolves the undesired isomers while the compound of Formula IV (A) is partially or completely insoluble in said solvent.

15. A process according to claim 14, wherein the solvent is a C₁₋₃ alkanol or an aliphatic ester, or a mixture thereof

16. A process according to claim 15, wherein the solvent is methanol or isopropyl acetate, or a mixture thereof.

## Patentansprüche

1. Verfahren zur Herstellung eines substituierten 1,3-Oxathiolans der Formel I oder seiner Stereoisomere und deren Salze, 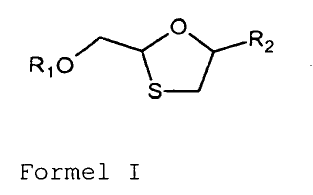 worin R₁ Wasserstoff, Alkyl oder Aryl ist und R₂ eine substituierte oder unsubstituierte Purin- oder Pyrimidinbase ist, die aus der aus 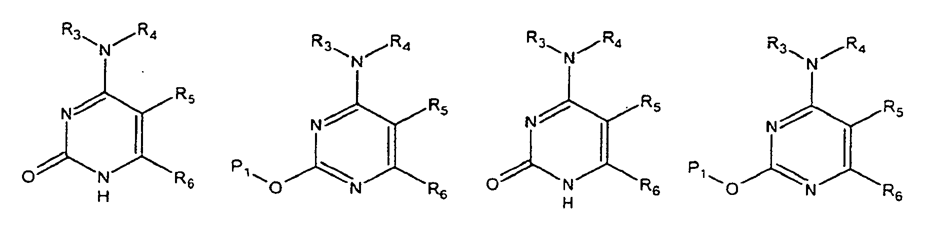 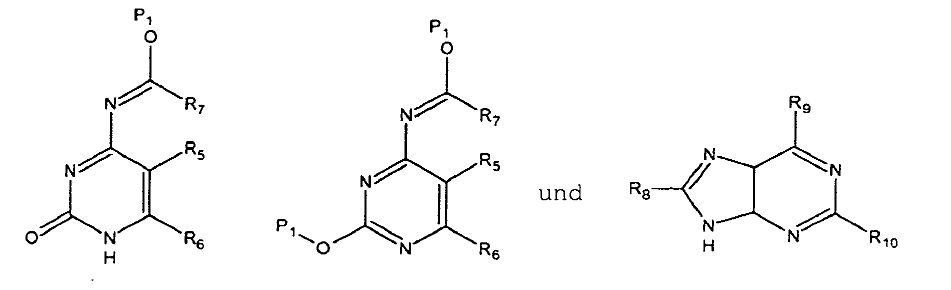 bestehenden Gruppe ausgewählt ist, wobei R₁ eine Schutzgruppe ist; R₃ und R₄ unabhängig voneinander aus der aus Wasserstoff, Hydroxyl, Amin und substituiertem oder und substituiertem C₁₋₆-Alkyl, C₁₋₆-Alkenyl, C₁₋₆-Alkinyl, C₁₋₆-Acyl oder Aracyl bestehenden Gruppe ausgewählt sind; R₅ und R₆ unabhängig voneinander aus der aus Wasserstoff, Halogen, Hydroxyl, Amin, Cyan, Carboxyl, Carbamoyl, Alkoxycarbonyl, Hydroxymethyl, Trifluormethyl, Thioaryl und substituiertem oder unsubstituiertem C₁₋₆-Alkyl, C₁₋₆-Alkinyl, C₁₋₆-Alkinyl oder C₁₋₁₀-Acyloxy bestehenden Gruppe ausgewählt sind; R₇ C₁₋₆-Alkyl, C₁₋₆-Alkenyl oder C₁₋₆-Alkinyl ist; R₈ aus der aus Wasserstoff, Hydroxyl, Alkoxy, Thiol, Thioalkyl, substituiertem oder unsubstituiertem Amin, Halogen, Cyan, Carboxyl, Alkoxycarbonyl, Carbamoyl und substituiertem oder unsubstituiertem C₁₋₆-Alkyl, C₁₋₆-Alkenyl, C₁₋₆-Alkinyl oder C₁₋₁₀-Acyloxy bestehenden Gruppe ausgewählt ist; und R₉ und R₁₀ aus der aus Wasserstoff, Hydroxyl, Alkoxy, substituiertem oder unsubstituiertem Amin, Halogen, Azido und substituiertem oder unsubstituiertem C₁₋₆-Alkyl, C₁₋₆-Alkenyl, C₁₋₆-Alkinyl oder C₁₋₁₀-Acyloxy bestehenden Gruppe ausgewählt sind, wobei das Verfahren umfasst
a) Umsetzung einer Verbindung der Formel III oder ihrer Stereoisomeren 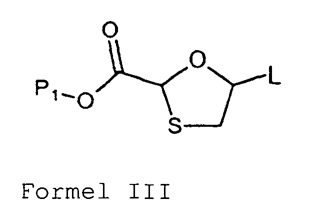 worin P₁ Wasserstoff oder eine Schutzgruppe und L eine Abgangsgruppe ist, mit einer wie oben definierten substituierten oder unsubstituierten Purin- oder Pyrimidinbase in Gegenwart einer Lewissäure mit der Maßgabe, dass die Lewissäure keine Silylgruppen enthält, zur Gewinnung einer Verbindung der Formel IV oder ihrer Stereoisomere, 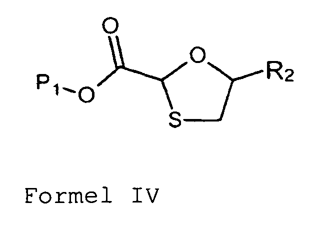 worin P₁ Wasserstoff oder eine Schutzgruppe ist und R₂ eine wie oben definierte substituierte oder unsubstituierte Purin- oder Pyrimidinbase ist,
b) Reduktion der Verbindung der Formel IV oder ihrer Stereoisomeren zur Gewinnung der Verbindung der Formel I oder ihrer Stereoisomeren und
c) Isolierung der Verbindung der Formel I oder ihrer Stereoisomeren und deren Salze aus deren Reaktionsgemisch.

2. Verfahren nach Anspruch 1, wobei die Lewissäure Zinn(IV)-chlorid oder Titantetrachlorid ist.

3. Verfahren nach Anspruch 2, worin die Lewissäure Zinn(IV)-chlorid ist.

4. Verfahren nach Anspruch 1, worin Stufe b) unter Verwendung eines Reduktionsmittels ausgeführt wird.

5. Verfahren nach Anspruch 1, worin die Verbindung der Formel I Lamivudin ist.

6. Verfahren zur Herstellung von Lamivudin der Formel I(A) oder einer Verbindung der Formel I(C) oder von Gemischen aus diesen 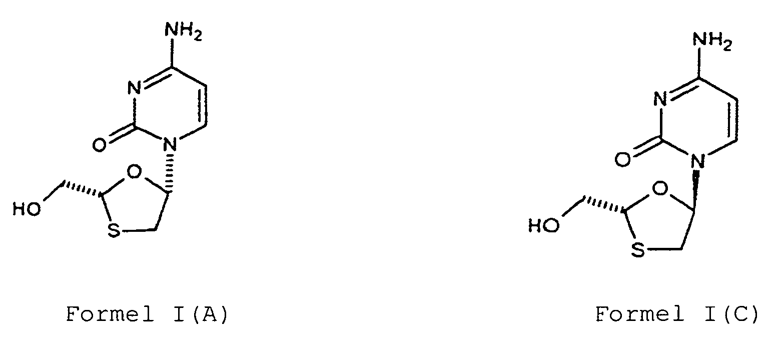 wobei das Verfahren umfasst
a) Umsetzen einer Verbindung der Formel III(A) oder Formel III (B) 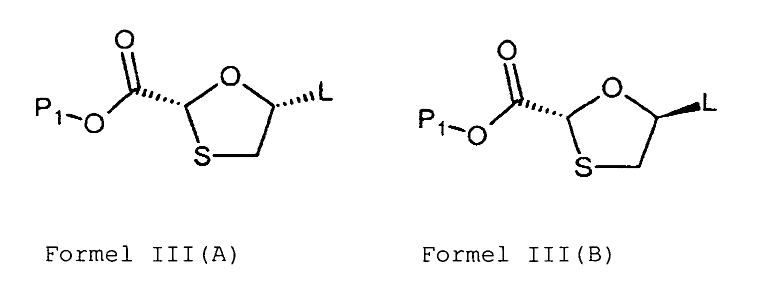 worin P₁ eine chirale Hilfsgruppe und L eine Abgangsgruppe ist, mit Cytosin, wobei die Amino- oder Hydroxylgruppe oder beide Gruppen des Cytosins ungeschützt oder mit Schutzgruppen geschützt sind, in Gegenwart einer Lewissäure mit der Maßgabe, dass die Lewissäure keine Silylgruppe enthält, zur Gewinnung einer Verbindung der Formel IV(A) oder Formel IV(B) oder Gemischen aus diesen 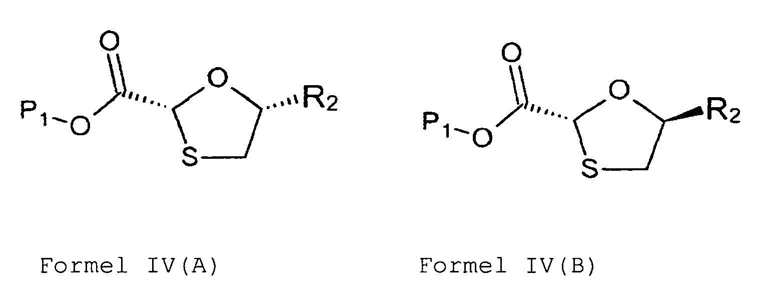 worin P₁ eine chirale Hilfsgruppe und R₂ Cytosin ist, worin die Amino- oder Hydroxylgruppe oder beide Gruppen des Cytosins gegebenenfalls mit Schutzgruppen geschützt sind,
b) Reduzieren der Verbindung der Formel IV(A) oder Formel IV(B) oder Gemischen aus diesen zur Gewinnung von Lamivudin der Formel I(A) oder der Verbindung der Formel I(C) oder Gemischen aus diesen, und
c) Isolieren des Lamivudins der Formel I(A) oder der Verbindung der Formel I(C) oder eines Gemisches aus diesen aus dem Reaktionsgemisch.

7. Verfahren nach Anspruch 6, worin die chirale Hilfsgruppe P₁ der Verbindung der Formel III(A) oder Formel III(B) oder von Gemischen aus diesen, und jener der Verbindung der Formel IV(A) oder Formel IV(B) oder von Gemischen aus diesen eine L-Menthylgruppe ist.

8. Verfahren nach Anspruch 6, worin das Cytosin mit Acetyl- und/oder Silylschutzgruppen geschützt ist.

9. Verfahren nach Anspruch 6, worin die Lewissäure Zinn(IV)-chlorid oder Titantetrachlorid ist.

10. Verfahren nach Anspruch 9, worin die Lewissäure Zinn(IV)-chlorid ist.

11. Verfahren zur Herstellung von Lamivudin der Formel I(A) 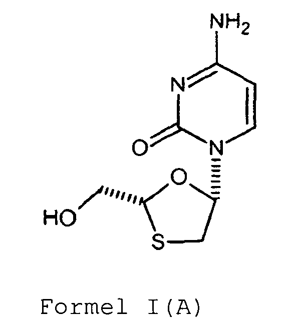 wobei das Verfahren umfasst
a) Umsetzung einer Verbindung der Formel III(C) 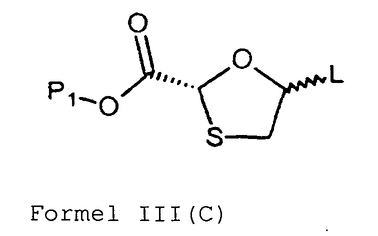 worin P₁ eine chirale Hilfsgruppe und L eine Abgangsgruppe ist, mit Cytosin, wobei die Amino- oder Hydroxylgruppe oder beide Gruppen des Cytosins ungeschützt oder mit Schutzgruppen geschützt sind, in Gegenwart einer Lewissäure mit der Maßgabe, dass die Lewissäure keine Silylgruppe enthält, zur Gewinnung einer Verbindung der Formel IV(C), 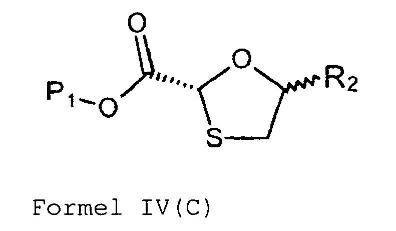 worin P₁ eine chirale Hilfsgruppe und R₂ Cytosin ist, worin die Amino- oder Hydroxylgruppe oder beide Gruppen des Cytosins ungeschützt oder mit Schutzgruppen geschützt sind,
b) Abtrennung einer Verbindung der Formel IV(A) aus dem Reaktionsgemisch, 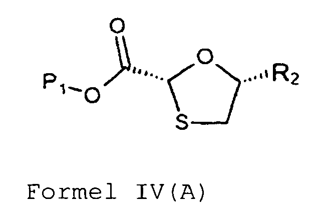 worin P₁ eine chirale Hilfsgruppe und R₂ Cytosin ist, worin die Amino- oder Hydroxylgruppe oder beide Gruppen des Cytosins ungeschützt oder mit Schutzgruppen geschützt sind,
c) Reduzieren der Verbindung der Formel IV(A) und
d) Isolieren von Lamivudin der Formel I(A) aus dem Reaktionsgemisch.

12. Verfahren nach Anspruch 11, worin die Lewissäure Zinn(IV)-chlorid oder Titantetrachlorid ist.

13. Verfahren nach Anspruch 12, worin die Lewissäure Zinn(IV)-chlorid ist.

14. Verfahren nach Anspruch 11, worin die Verbindung der Formel IV(A) im Schritt b) durch Behandeln der Verbindung der Formel IV(C) mit einem Lösemittel, das die unerwünschten Isomeren selektiv löst, während die Verbindung der Formel IV(A) teilweise oder völlig in diesem Lösungsmittel unlöslich ist, abgetrennt wird.

15. Verfahren nach Anspruch 14, worin das Lösemittel ein C₁₋₃-Alkanol oder ein aliphatischer Ester oder ein Gemisch aus diesen ist.

16. Verfahren nach Anspruch 15, worin das Lösemittel Methanol oder Isopropylacetat oder ein Gemisch aus diesen ist.

## Revendications

1. Procédé de préparation d'un 1,3-oxathiolane substitué de formule I ou de ses stéréoisomères et de ses sels, 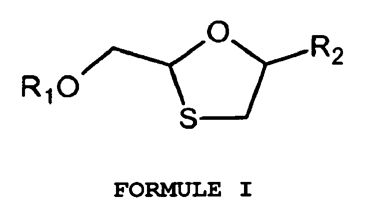 dans laquelle R₁ est de l'hydrogène, un groupement alkyle ou aryle, et R₂ est une base de purine ou de pyrimidine substituée ou non substituée choisie dans le groupe constitué des suivantes : 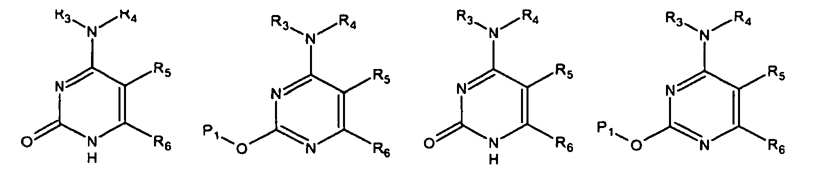 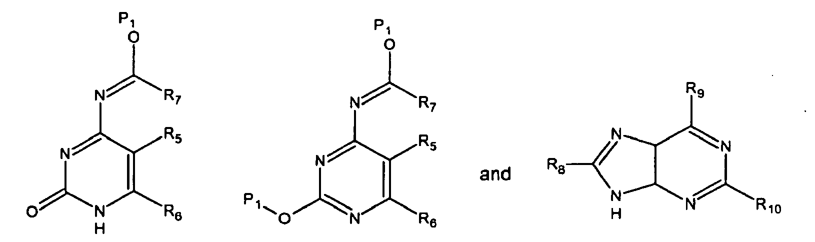 dans lesquelles P₁ est un groupement protecteur, R₃ et R₄ sont indépendamment choisis dans le groupe constitué de l'hydrogène, d'un groupement hydroxyle, amino et alkyle en C₁₋₆, alcényle en C₁₋₆, alcynyle en C₁₋₆, acyle ou aracyle en C₁₋₆ substitués ou non substitués ; R₅ et R₆ sont indépendamment choisis dans le groupe constitué de l'hydrogène, d'un halogène, d'un groupement hydroxyle, amino, cyano, carboxy, carbamoyle, alcoxycarbonyle, hydroxyméthyle, trifluorométhyle, thioaryle et les groupements alkyle en C₁₋₆, alcényle en C₁₋₆, alcynyle en C₁₋₆ ou acyloxy en C₁₋₁₀ substitués ou non substitués ; R₇ est un groupement alkyle en C₁₋₆, alcényle en C₁₋₆ ou alcynyle en C₁₋₆ ; R₈ est choisi dans le groupe constitué de l'hydrogène, d'un groupement hydroxy, alcoxy, thiol, thioalkyle, amino substitué ou non substitué, d'un halogène, d'un groupement cyano, carboxy, alcoxycarbonyle, carbamoyle et d'un groupement alkyle en C₁₋₆, alcényle en C₁₋₆, alcynyle en C₁₋₆ ou acyloxy en C₁₋₁₀ substitués ou non substitués ; et R₉ et R₁₀ sont choisis dans le groupe constitué de l'hydrogène, d'un groupement hydroxy, alvoxy, amino substitué ou non substitué, d'un halogène, d'un groupement azido et d'un groupement alkyle en C₁₋₆, alcényle en C₁₋₆, alcynyle en C₁₋₆ ou acyloxy en C₁₋₁₀ substitués ou non substitués, dans lequel le procédé comprend les étapes consistant à :
a) faire réagir un composé de formule III ou ses stéréoisomères, 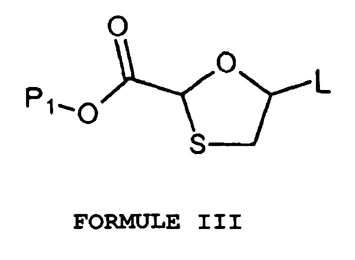 dans laquelle P₁ est de l'hydrogène ou un groupement protecteur et L est un groupe quittant, avec une base de purine ou de pyrimidine substituée ou non substituée définie ci-dessus, en présence d'un acide de Lewis à condition que l'acide de Lewis ne contienne pas de groupement silyle quelconque, pour obtenir un composé de formule IV ou ses stéréoisomères, 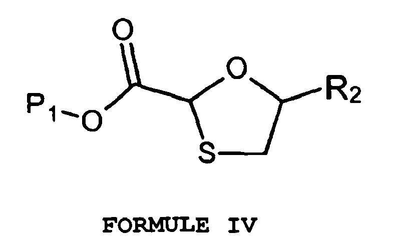 dans laquelle P₁ est de l'hydrogène ou un groupement protecteur et R₂ est une base de purine ou de pyrimidine substituée ou non substituée,
b) réduire le composé de formule IV ou ses stéréoisomères et
c) isoler le composé de formule I ou ses stéréoisomères et ses sels de son mélange réactionnel.

2. Procédé selon la revendication 1, dans lequel l'acide de Lewis est le chlorure stannique ou le tétrachlorure de titane.

3. Procédé selon la revendication 2, dans lequel l'acide de Lewis est le chlorure stannique.

4. Procédé selon la revendication 1, dans lequel l'étape b) est effectuée en utilisant un agent réducteur.

5. Procédé selon la revendication 1, dans lequel le composé de formule I est la lamivudine.

6. Procédé de préparation de lamivudine de formule I(A) ou d'un composé de formule I(C) ou d'un de leurs mélanges : 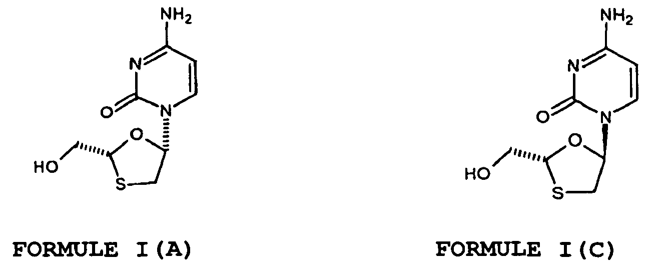 dans lequel le procédé comprend les étapes consistant à :
a) faire réagir un composé de formule III(A) ou de formule III(B) ou de leurs mélanges, 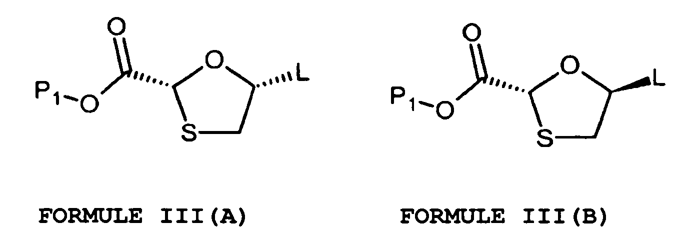 dans lesquelles P₁ est un auxiliaire chiral et L est un groupe quittant, avec de la cytosine, dans laquelle le groupement amino ou hydroxy ou les deux groupements de ladite cytosine sont non protégés ou protégés par des groupements protecteurs en présence d'un acide de Lewis à condition que l'acide de Lewis ne contienne pas de groupement silyle quelconque, pour obtenir un composé de formule IV(A) ou de formule IV(B), ou un de leurs mélanges, 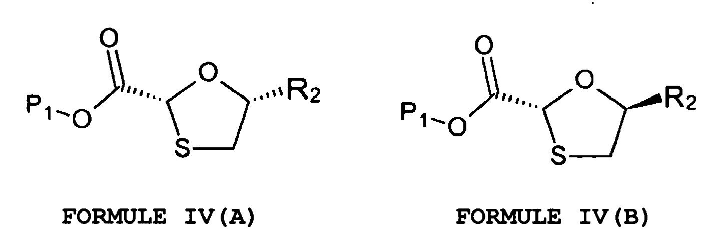 dans lesquelles P₁ est un auxiliaire chiral et R₂ est la cytosine, dans laquelle le groupement amino ou hydroxy ou les deux groupements de ladite cytosine sont facultativement protégés par des groupements protecteurs,
b) réduire le composé de formule IV(A) ou de formule IV(B) ou leurs mélanges, pour obtenir de la lamivudine de formule I(A) ou le composé de formule I(C), ou leurs mélanges, et
c) isoler la lamivudine de formule I(A) ou le composé de formule I(C), ou leurs mélanges, du mélange réactionnel.

7. Procédé selon la revendication 6, dans lequel l'auxiliaire chiral P₁ du composé de formule III(A) ou de formule III(B) ou de leurs mélanges, et celui du composé de formule IV(A) ou de formule IV(B), ou de leurs mélanges, sont un groupement L-menthyle.

8. Procédé selon la revendication 6, dans lequel la cytosine est protégée par des groupements protecteurs acétyle et/ou silyle.

9. Procédé selon la revendication 6, dans lequel l'acide de Lewis est le chlorure stannique ou le tétrachlorure de titane.

10. Procédé selon la revendication 9, dans lequel l'acide de Lewis est le chlorure stannique.

11. Procédé de préparation de lamivudine de formule I(A), 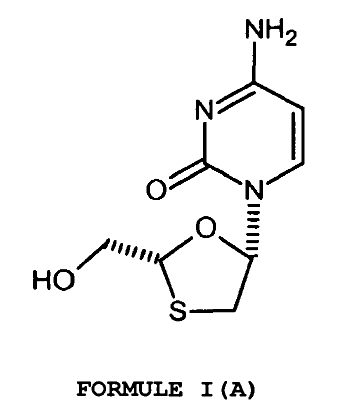 dans laquelle le procédé comprend les étapes consistant à :
a) faire réagir un composé de formule III (C), 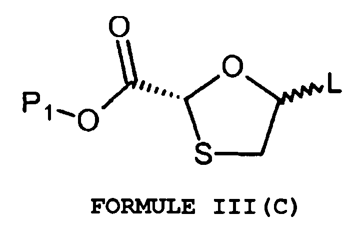 dans laquelle P₁ est un auxiliaire chiral et L est un groupement quittant, avec de la cytosine, dans laquelle le groupement amino ou hydroxy ou les deux groupements de ladite cytosine sont non protégés ou protégés par des groupements protecteurs, en présence d'un acide de Lewis à condition que l'acide de Lewis ne contienne pas de groupement silyle quelconque, pour obtenir un composé de formule IV(C), 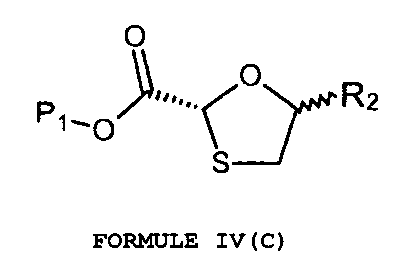 dans laquelle P₁ est un auxiliaire chiral et R₂ est de la cytosine, dans laquelle le groupement amino ou hydroxy ou les deux groupements de ladite cytosine sont non protégés ou protégés par des groupements protecteurs,
b) séparer un composé de formule IV(A) de son mélange réactionnel, 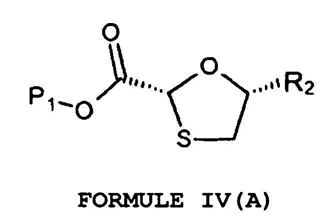 dans laquelle P₁ est un auxiliaire chiral et R₂ est de la cytosine, dans laquelle le groupement amino ou hydroxy ou les deux groupements de ladite cytosine sont non protégés ou protégés par des groupements protecteurs,
c) réduire le composé de formule IV(A) et
d) isoler la lamivudine de formule I(A) de son mélange réactionnel.

12. Procédé selon la revendication 11, dans lequel l'acide de Lewis est le chlorure stannique ou le tétrachlorure de titane.

13. Procédé selon la revendication 12, dans lequel l'acide de Lewis est le chlorure stannique.

14. Procédé selon la revendication 11, dans lequel le composé de formule IV(A) est séparé à l'étape b) en traitant le composé de formule IV(C) avec un solvant qui dissout sélectivement les isomères non souhaités, tandis que le composé de formule IV(A) est partiellement ou complètement insoluble dans le solvant.

15. Procédé selon la revendication 14, dans lequel le solvant est un alcanol en C₁₋₃ ou un ester aliphatique ou un de leurs mélanges.

16. Procédé selon la revendication 15, dans lequel le solvant est le méthanol ou l'acétate d'isopropyle ou un de leurs mélanges.
